# EUROPEAN PATENT APPLICATION

(11) **EP 3 628 316 A1**
(43) Date of publication of application: **01.04.2020**
(21) Application number: 17902824.6
(22) Date of filing: 30.03.2017
(51) Int. Cl.: A61K 31/19, A61K 31/375, A61K 31/51, A61K 31/714, A61K 33/06

(54) **MULTIVITAMIN COMPOSITION FOR IMPROVING VERBAL FLUENCY, DECREASING PERFORMANCE ANXIETY SYMPTOMS AND METHOD FOR PREPARING SAME**

(71) Applicant: Penello Temporão, José Eduardo, 22460-200 Rio de Janeiro - RJ (BR)
(72) Inventor: Penello Temporão, José Eduardo, 22460-200 Rio de Janeiro - RJ (BR)
(74) Representative: Temiño Ceniceros, Ignacio
(86) International application number: PCT/BR2017/000025
(87) International publication number: WO 2018/176109

(57) **Abstract**

The present invention applies in the field of compositions and supplements and multivitamins. The present invention discloses a multivitamin composition which provides an improvement in speech fluency and the performance anxiety control, when administered to patients in therapeutically effective amounts.

## Description

### FIELD OF THE APPLICATION

The present invention applies in the field of compositions and supplements and multivitamins.

The present invention discloses a multivitamin composition, presenting Vitamin B1 (thiamine), Theanine and Magnesium as main elements, as well as other Vitamins, amino acids and excipients, which provide an improvement in verbal fluency and the control of performance anxiety symptoms. When administered to patients in therapeutically effective amounts (orally via pills, tablets, chewing gums, candies or transdermically) it brings direct benefits in the improvement of the verbal fluency dysfunctions as well as performance anxiety symptoms.

### BACKGROUND OF THE INVENTION

The verbal fluency dysfunctions, such as, for example, stuttering, which is an interruption in verbal fluency which can be characterized by sound repetitions or extensions, in general, does not have control and is associated to the development of several other diseases and symptoms, such as depression, anxiety disorders, mood disorders and performance anxiety.

The verbal fluency dysfunctions themselves are known for many centuries and occur in all ethnicities and social classes. However, up to the moment, different approaches from the alternatives broadly used (psychotherapy, speech therapy, etc.) are extremely rare, even though they present excellent results, with low cost and no side effects.

The verbal fluency dysfunctions can be considered as a public health problem, since they incapacitate the individuals with such dysfunction in a way to chronically limit their abilities of developing professional and affective relations. A drug has not yet been approved for the treatment of any of these disorders, and the individuals with such disorders are totally orphans of a drug treatment and totally dependent on expensive solutions, long lasting and low efficacy, such as psychotherapy and speech therapy.

Some theories indicate that a probable cause for the major dysfunction of verbal fluency, the stuttering, is a structural or functional abnormality of the central nervous system.

When we enter the field of vitamins, minerals and amino acids, exploring their great therapeutic capacities and going in the opposite way from the traditional medical research, we have found that there are natural ways to reduce and even eliminate some of the symptoms that afflict the millions of people with these disorders worldwide.

The multivitamin composition claimed herein aims to improve the speech fluency, in people of all kinds and from 5 years old, with or without such disorders, and help in the performance anxiety control, using the synergy of the active pharmaceutical ingredients described, such as vitamins, minerals and amino acids, creating an unique and innovative effect.

### STATE OF THE ART

Brazilian document PI 0607684-0 discloses a pharmaceutical composition comprising compound (5)- N- [2- (1,6,7,8- tetrahydro- 2- indene [5,4- b] furan- 8-yl)- ethyl]- propionamide, besides other drugs. This document is different from the composition of the present invention because it does not present any antidepressant drug class disclosed by the present invention.

American document US 2016271141 discloses treatment methods for an individual who stutters, comprising the administration of a composition comprising D1 / D5 receptors or mixtures thereof. The present invention differs from this document, since it uses different ingredients in its composition and not D1 / D5 receptors.

American document US 7070804 discloses a tablet with greater compatibility by human beings comprising: (A) at least one Vitamin, (B) optionally at least one mineral, (C) Lysine or a pharmaceutically acceptable salt thereof, (D) at least one sweetener and optionally at least one flavoring agent which presents the capacity of masking the flavor of Lysine, (E) and a pharmaceutically or dietetically acceptable vehicle. Such document discloses some components known from the state of the art, however, at no point does it mention the application in the improvement of verbal fluency dysfunctions and control of the performance anxiety symptoms.

### SUMMARY OF THE INVENTION

The present invention discloses a multivitamin composition developed to potentialize and improve the speech fluency, as well as control the performance anxiety symptoms, providing a feeling of well-being, having the following components: Vitamin B1, Magnesium, Lysine, Theanine, Vitamin C and Vitamin B12.

### DETAILED DESCRIPTION OF THE INVENTION

The voice is the main mean of communication between human beings and between human beings and animals. As several speech therapists attest, it is almost impossible for someone to have a perfect fluency in speech, even in healthy people. The present invention discloses a multivitamin composition which improves speech fluency by introducing active pharmaceutical ingredients, such as Vitamins, minerals and amino acids which act on the central nervous system and in the cerebral mechanisms of speech, improving verbal fluency and decreasing the symptoms caused by these disorders.

The performance anxiety, also known as stage fright, affects children and adults, men and women and characterizes by a set of physiological, psychological and behavioral reactions when the individual is exposed to stressful events, such as sports competitions, evaluations in academic environments, artistic presentations, among other activities. The present invention herein disclosed was developed to also ameliorate and relieve physical, psychological and behavioral symptoms of the speech in stressful moments and anxiety in adults and children.

The synergy of the elements of the present invention, in their dosages herein pre-established, creates un unique and totally innovative effect, by presenting efficiency in the improvement of the speech fluency, control of the performance anxiety symptoms and by bringing a sense of calm and well-being, using a natural formulation and with no side effects.

The present invention discloses a multivitamin composition developed to potentialize and improve the speech fluency and control the performance anxiety symptoms, presenting from 17 % to 35 % of Vitamin B1 or Thiamine Hydrochloride, from 5 % to 9 % of Magnesium or Magnesium Glycinate, from 8 % to 17 % of Lysine or Lysine Hydrochloride, from 4 % to 13 % of Theanine or L-Theanine, from 8 % to 26 % of Vitamin C or ascorbic acid, from 0,05 % to 0,9 % of Vitamin B12 or methyl cobalamin and excipients.

In an embodiment, the composition of the present invention comprises active ingredients from 40 % to 70 % of total of the composition and excipients from 30 % to 60 % of the total of the composition.

Vitamin B1, or thiamine, was the first Vitamin from B complex to be identified. Its function in the organism is in the form of coenzyme thiamine pyrophosphate (TPP) and its transformation in coenzyme is performed by the action of adenosine triphosphate (ATP) as donor of pyrophosphate (PP).

Such Vitamin acts on the metabolism of carbohydrates as a coenzyme in the decarboxylation of α-ketoacids and transformation of pentose to hexose monophosphate with energy production.

The literature shows that the intake of high doses of thiamine is linked to increased learning ability, decreased stress and the prevention of memory loss, including in patients with Alzheimer's disease.

However, in view of the little interest shown by pharmaceutical companies and by the by academics themselves in using natural substances to treat verbal fluency dysfunctions, mainly stuttering individuals, only one scientific study of this kind was carried out. "A Consideration of Thiamin Supplement in Prevention of Stuttering in Preschool Children", by American doctor Lester L Hale, published in 1951, pointed out, even preliminary, that only the Vitamin B1 (thiamine) intake by stuttering children would bring benefits to these individuals.

In tests with the proposed formulation in the present invention, the combination between Vitamin B1, the Theanine and the Magnesium, in the dosages herein established and together with the other components, presented a potent and incisive effect in individuals with speech fluency disorders ranging from individuals with mild changes to individuals with chronic changes in verbal fluency.

The Vitamin C, or ascorbic acid, acts as an antioxidative and, in tissue level, acts as a cofactor to several enzymes involved in the biosynthesis of the collagen, carnitine and neurotransmitters, proteoglycans and other organic constituents of the intercellular matrix of several tissues such as teeth, bones and endothelium.

Its antioxidant action acts mainly on the protection against free radicals, protection against cataracts formation and degeneration of the macula in elderly patients.

Vitamin C is necessary to the ideal activity of several important biosynthetic enzymes and, thus, it is essential for several metabolic pathways in the body. This Vitamin also acts as an efficient eliminator of oxidizing radicals, thus, protecting other biomolecules from oxidative damage. In addition, Vitamin C can save or recycle glutathione and Vitamin E, two other important physiological antioxidants.

In addition, Vitamin C has a modulating effect on catecholaminergic activity, in particular, the conversion of dopamine to norepinephrine, decreasing stress reactivity, depression, hypochondria, mood changes, prolactin release, improves vascular function and increases the oxytocin release. These processes are relevant to sexual and mood behavior, disorders that most often affect people with communication difficulties. Studies have shown that the administration of Vitamin C in moderate dosages significantly reduces mood disorders.

When administered together with conventional antidepressants, Vitamin C acts producing a synergistic effect as a coadjutant for the treatment of depression, another extremely common disease in individuals with disorders in speech fluency.

The Vitamin B12, or methyl cobalamin, acts as a coenzyme in several metabolic functions of fats, carbohydrates and synthesis of proteins, also participating in the formation of red blood cells by activating folic acid coenzymes.

Vitamin B12 is easily absorbed in the intestinal tract in the lower half of the ileum. The presence of calcium and intrinsic factor are indispensable for its absorption and, once the Vitamin is absorbed, it binds to plasma proteins.

The lack of Vitamin B12 also interferes with the folic acid mechanism, causing hematological abnormalities.

Vitamin B12 deficiency has significative impact on the nervous system causing irreversible lesions, with progressive increase of the myelinated neurons, demyelination and death of neuronal cells in the spine and cerebral cortex, with symptoms such as paresthesia of the hands and feet, decreased sensation of vibration and position, with consequent instability, confusion, moodiness and loss of memory and central vision, possibly still exhibiting delirium, hallucinations or even frank psychosis.

Recent studies with depressive patients show a low level of Vitamin B12 and the best results in the treatment was found when the Vitamin is administered in greater doses. Recent literatures proved the relationship between Vitamin B12 deficiency and depression. It is common among individuals with depression to have poor responses to antidepressant treatment compared to vitamin B12 treatment.

Other studies show that the association of Vitamin B12 with other micronutrients, mainly when associated to Magnesium salts, one of the components of the present invention, showed rapid recovery from patients with symptoms of depression.

When combining these two elements - Vitamin B12 and Magnesium - in the dosages stablished by the present invention, a direct result in the feeling of well-being of the tested individuals is obtained, compared to the placebo group.

The L-Theanine, or gamma-ethyl amide L-glutamic acid or 5-n-ethyl-L-glutamine, is an amino acid analogous to glutamic acid. This compound acts on the body by increasing the production of gamma amino butyl acid (GABA) by altering serotonin and dopamine levels in the brain.

As an analogue to excitatory glutamate neurotransmitters, Theanine is capable of binding to glutamate receptors and transmitters. Receptors are found in synaptic cells and are responsible for transmitting excitatory signals. Transmitters are found in neural and glical cells, removing glutamate from the extracellular synaptic space helping to regulate glutamate level and because of neural signaling.

Its absorption occurs at brain level, being easy to cross the blood brain barrier, which is mediated by leucine, increasing the alpha brain waves and presenting stimulating psychoactive properties with reducing effect on physical and mental stress, improving cognition and mood, also contributing to improving memory and learning processes.

Randomized, double-blind, placebo-controlled studies in patients with schizophrenic disorders have shown that increased L-Theanine antipsychotic therapy reduces positive activation and anxiety symptoms.

Studies show that L-Theanine intake provides an increased brain oscillatory activity in the so-called alpha range (8 - 14 Hz) during resting EEG records in human beings. Concomitantly, the alpha range activity has been shown to be a key component in selective attention performance while performing mental tasks.

It has a marked anxiolytic and soothing effect due to its inhibitory neurotransmitter regulation property and modulation of serotonin and dopamine.

Other studies showed that L-Theanine intake resulted in a reduction in heart rate and salivary immunoglobulin A responses in individuals undergoing an acute stress task in relation to the placebo control condition. These effects were attributable to attenuation of sympathetic nerve activation with anti-stress effects by inhibiting cortical neuronal excitation.

Due to its potent anxiolytic effect, L-Theanine acts directly on one of the biggest problems of individuals with speech fluency disorders: anxiety.

Anxiety not only significantly worsen verbal fluency, but it also has devastating physical and psychological effects on individuals who need to perform tasks in public, such as giving a lecture, giving a class or even attending a theater performance. For this kind of anxiety, which comes on the verge of performing tasks in public, we call performance anxiety.

L-Lysine is an essential amino acid involved in the synthesis of proteins in the organism, being necessary, together with other amino acids, to growing and tissue repair.

It has anxiolytic action through its effects on serotonin receptors and reduces anxiety by regulating serotonin in the amygdala, also acting as a benzodiazepine agonist.

Studies have positively evaluated Lysine's ability to block the effects of stress and reduce anxiety. Results from a double-blind, randomized, placebo-controlled study confirm the modulating effect of Lysine treatment in healthy individuals with high subjective levels of mental disorder, stress and chronic anxiety, common symptoms in people with verbal fluency dysfunction.

In trials performed by using the composition of the present invention, the L-Lysine has become an essential component and has been shown to have a positive effect on anxiety amelioration and, consequently, on improvement in speech fluency, as well as, together with the other substances, induce a feeling of well-being in individuals with performance anxiety.

As regards Magnesium, its mechanism of action is related to ion exchange activity and osmotic activity.

Magnesium is the forth mineral in greater abundance in the human organism and is involved in about 325 enzymatic reactions, including cellular energy production via ATP-Mg complex formation, cell membrane stabilization and nucleic acid, proteins and cytoplasmic organelles synthesis. Therefore, this mineral plays an important role in the electrical stability and integrity of the cell membrane, muscle contraction, nerve conduction, regulation of vascular tone, among others. It has also been associated with anxiety-related disorders.

Deficiency (hypomagnesemia) causes nervous system irritability with tetany, fatigue, vasodilation, muscle spasms or cramps, convulsions, tremors, depression and psychotic behavior.

Studies show that the association of Vitamin B12 with other micronutrients, especially when associated with Magnesium salts (glycinate or taurinate), showed rapid recovery in patients with symptoms of depression.

Sixty per cent of the clinic depression cases are considered treatment resistant depression. The Magnesium deficiency causes the N-methyl-D-aspartate-coupled calcium channels to tilt into the opening, causing neuronal injury and neurological dysfunction, which may appear as major depression. Magnesium administration led to antidepressant-like effects that were comparable to those of strong antidepressants. Magnesium in cerebrospinal fluid was considered low in treatment-resistant suicidal depression and in patients who have attempted suicide.

Randomized clinical trials have shown that Magnesium is as effective as the tricyclic antidepressant imipramine in treating depression in diabetics and without any of the side effects of imipramine.

Intravenous and oral Magnesium, in specific protocols, it has been reported to quickly treat depression safely and without side effects. Studies show that cerebral Magnesium insufficiency can reduce serotonin levels, as antidepressants have been shown to increase cerebral Magnesium, an additional hypothesis that Magnesium treatment is beneficial for almost all depressed people.

Studies performed by using the Hospital Anxiety and Depression Scale have shown that standardized Magnesium intake has a close relation in reducing the symptoms of depression and anxiety in the community and consequently health policy implications.

In addition, Vitamin B1 depends on Magnesium to "be functional". Without the proper amount of Magnesium, Vitamin B1 is not capable of perform essential tasks in the central nervous system. Our studies in stuttering individuals concluded that the administration of the Magnesium together with Vitamin B1 presented better results when compared to the administration of only Vitamin B1.

For its crucial role in over 400 body mechanisms, Magnesium, together with other components presented on the present invention, acts as a capacitator, allowing that all substances be properly absorbed and delivered, correcting possible deficiencies which result in harmful effects to the speech fluency, performance anxiety and the sensation of malaise related to those sickness.

Based on the studies performed with the pharmaceutical ingredients disclosed, evidenced mainly by the actions on the nervous system, especially in the relation of depression disorders and anxiety disorders, which are closely related to speech disorders, the synergy of their use when used together and under the conditions of this invention, demonstrated a considerable potentialization of its effects, being benefic and not presenting side effects in individuals having speech disfunction and / or performance anxiety.

Some examples of excipients used in the composition of the present invention are monohydrated lactose, microcrystalline cellulose, starch, polyvinylpyrrolidone, sodium croscarmellose, calcium stearate, but not limited to the same.

Such components of the present composition are obtained from the market of manufacturers and suppliers accredited by the competent official bodies, and they have assured quality and they are in compliance with the official compendia, mainly the Brazilian and American pharmacopoeias.

The term "active pharmaceutical ingredient" refers to any substance or substance blend intended to be used in the manufacture of a pharmaceutical formulation and, when used in this form, it becomes an active ingredient of the pharmaceutical form. These substances are intended to provide pharmacological activity or other direct effect in the diagnosis, cure, relief, treatment or prevention of an illness or to affect the structure and functioning of the body.

The term "excipient" refers to any substance other than active pharmaceutical ingredients that has been properly assessed for its safety and which is included in an active release system to assist in the processing of the active release system during manufacture; protect, maintain or increase stability, bioavailability or patient acceptance; assist in asset identification; and / or improve the quality of another general attribute of safety and efficacy of the pharmaceutical product during its storage or use.

The term "therapeutically effective amount" refers to an amount which is efficient in reducing, eliminating, treating, preventing or controlling the symptoms of the diseases and conditions herein described.

The term "control" is meant to refer to all processes in which there may be a slowing, interruption, cessation or interruption of progression of the diseases and conditions described herein, but does not necessarily indicate a total elimination of all symptoms of the disease.

The term "single dose" means a single dose which is capable of being administered in an individual and which can be easily handled and packaged, remaining as a single dose physically and chemically stable comprising the pharmaceutically acceptable composition.

For the formulation of the composition of the present invention, it is used one or more of diluents, each one independently selected from a starch, a lactose monohydrate or a microcrystalline cellulose. One or more of disintegrating agents, each one independently selected from a pregelatinized starch or a cross-linked sodium carboxy methylcellulose; a binder and a lubricant, but not limited to the examples provided.

In another embodiment, the binder is a polyvinylpyrrolidone, and the lubricant is magnesium stearate. In another embodiment, the present invention uses a diluent, such as lactose; a second diluent, such as microcrystalline cellulose; a disintegrator, such as pregelatinized starch; a second disintegrator, such as a sodium croscarmellose and a binder, such as polyvinyl pyrrolidone, but not limited to the same.

The excipients are selected to ensure the release of active pharmaceutical ingredients at a convenient unit dosage and to optimize the cost, ease and feasibility of the manufacturing process. All excipients must have the quality described in official compendia, such as American, Brazilian, European, pharmacopoeias and they must also be inert, organoleptically acceptable and compatible with the active pharmaceutical ingredients. The excipients used in a formulation in tablets usually include diluting agents; binding agents; disintegrating agents; lubricants; non sticking agents; gliding agents, such as talc and silicon dioxide; wetting agents, such as alcohol, water, propylene glycol and glycerin; and surfactants, such as sodium lauryl sulfate and ammonium quaternary; colorant and pigments, such as those selected from the ones included in the list (FD & C) approved by the competent bodies; flavoring agents, such as orange flavor, glucose syrup, mannitol, lactose, sugar; sweeteners and flavor masks, but not limited to the same.

Diluents are typically added to the pharmaceutically active ingredients of the formulation to provide mass, fluidity and compression to the tablet. The most common diluent is lactose, which exists in different available forms to be used as a diluent and to promote a suitable formulation. Several types of lactose can be used such as spray dried monohydrated lactose, alpha-lactose monohydrate, anhydrous alpha-lactose, anhydrous beta-lactose and agglomerated lactose. Other diluents include sugars, such as compressible sugar, the excipient dextrose. A preferred diluent is lactose monohydrate. Other preferred diluents include microcrystalline cellulose and microfine cellulose. As diluents, they can include starch and starch derivatives. The starches include native starches obtained from wheat, corn, rice and potatoes. Other starches include pregelatinized starch and sodium starch glycolate. The starches and starch derivatives also act as disintegrating agents. Other diluents include inorganic salts, such as dibasic calcium phosphate, tribasic calcium phosphate and calcium sulfate. Such polyols as mannitol, sorbitol and xylitol can also serve as diluents. Many diluents also work as disintegrating agents and binders.

The disintegrating agents are included in formulations to fractionate in small particles of the active pharmaceutical ingredients and excipients that constitute the tablets facilitating the dissolution and increasing the bioavailability of the active ingredient. Starch derivatives, including cross-linked sodium salt of a starch carboxymethyl ether, such as sodium starch glycolate, are disintegrating agents commonly used. A preferred disintegrator is pregelatinized starch. Other preferred disintegrator is cross-linked sodium carboxymethyl cellulose, such as Sodium croscarmellose. Other disintegrating agents include cross-linked polyvinylpyrrolidone, such as Crospovidone and microcrystalline cellulose.

The binders are used as a wet granulation excipient to agglomerate the pharmaceutically active ingredient and the other excipients. The binders include cellulose derivatives such as microcrystalline cellulose, methylcellulose, sodium carboxy methylcellulose, hydroxypropyl methylcellulose, hydroxyethyl cellulose and hydroxypropyl cellulose. Other binding agents include polyvidone, polyvinylpyrrolidone, gelatin, natural gums, such as acacia, tragacanthin, guar and pectin, starch pulp, pregelatinized starch, sucrose, corn syrup, polyethylene glycols and sodium alginate, ammonium and calcium alginate, Magnesium and aluminum silicate, polyethylene glycols. A preferred binder is polyvinylpyrrolidone.

The lubricants are used in the formulation of tablets to avoid the adherence of the tablet to the faces of the punch and to reduce the friction during the compression phases. The lubricants include, typically, plant oils, such as corn oil, mineral oils, polyethylene glycols, such as PEG-4000 and PEG-6000, salts of stearic acid, such as calcium stearate and sodium stearyl fumarate, mineral salts, inorganic salts, such as sodium chloride, organic salts, such as sodium benzoate, sodium acetate and sodium oleate and polyvinyl alcohols. A preferred lubricant is calcium stearate.

The multivitamin composition herein claimed can be developed under many pharmaceutical forms, such as tablet, capsules, lozenges, solutions, suspensions, effervescent tablets, chewing gum, transdermal patches, the tablet being the preferred pharmaceutical form of the present invention, but not limited to the pharmaceutical forms herein disclosed.

In order to present significant results, the composition should preferably be taken daily, but preferably three times a day and together with the meals, in adult individuals, three times a day in children from 7 to 10 years old and three times a day in children from 4 to 6 years old, in therapeutically effective amounts, depending on the pharmaceutical form administered.

### ILLUSTRATIVE EXAMPLES

A preferred example of formulation for administration in adult individuals of the present invention presents the composition:

| Components | Amount |
|---|---|
| Thiamine hydrochloride (Vitamin B1) | 1200,0 mg |
| Magnesium Glycinate | 300,0 mg |
| Lysine Hydrochloride | 450,0 mg |
| L-Theanine | 600,0 mg |
| Ascorbic acid (Vitamin C) | 900,0 mg |
| Methyl cobalamin (Vitamin B12) | 3,0 mg |
| Daily dose | 3453,0 mg |

For significant results to be reported, the composition described above should preferably be taken daily, more preferably in three daily doses of 1151 mg for each administered dose for adult individuals, and together with the meals.

Another example of basic formulation of the present invention for administration in children between 7 and 10 years old presents the composition:

| Components | Amount |
|---|---|
| Thiamine hydrochloride (Vitamin B1) | 800,0 mg |
| Magnesium Glycinate | 200,0 mg |
| Lysine Hydrochloride | 300,0 mg |
| L-Theanine | 400,0 mg |
| Ascorbic acid (Vitamin C) | 600,0 mg |
| Methyl cobalamin (Vitamin B12) | 2,0 mg |
| Daily dose | 2302,0 mg |

For significant results to be reported, the composition described above should preferably be taken daily, more preferably in three daily doses of 767,33 mg for each administered dose for children from 7 to 10 years old, and together with the meals.

Another example of basic formulation of the present invention for administration in children between 4 and 6 years old presents the composition:

| Components | Amount |
|---|---|
| Thiamine hydrochloride (Vitamin B1) | 400,0 mg |
| Magnesium Glycinate | 100,0 mg |
| Lysine Hydrochloride | 150,0 mg |
| L-Theanine | 200,0 mg |
| Ascorbic acid (Vitamin C) | 300,0 mg |
| Methyl cobalamin (Vitamin B12) | 1,0 mg |
| Daily dose | 1151,0 mg |

For significant results to be reported, the composition described above should preferably be taken daily, more preferably in three daily doses of 383,66 mg for each administered dose for children from 4 to 6 years old, and together with the meals.

One example of the basic formulation of the tablet for the present invention presents the composition:

| Components | Amount |
|---|---|
| Thiamine hydrochloride (Vitamin B1) | 200,0 mg |
| Magnesium Glycinate | 50,0 mg |
| Lysine Hydrochloride | 75,0 mg |
| L-Theanine | 100,0 mg |
| Ascorbic acid (Vitamin C) | 150,0 mg |
| Methyl cobalamin (Vitamin B12) | 0,5 mg |
| Monohydrated lactose | 139,0 mg |
| Microcrystalline cellulose | 125,0 mg |
| Starch | 150,0 mg |
| Polyvinylpyrrolidone | 60,0 mg |
| Sodium croscarmellose | 40,0 mg |
| Calcium stearate | 10,5 mg |
| Total weight of the tablet | 1100,0 mg |

In general, the pharmaceutical technological process developed for the preparation of the blend to be compressed into oblong tablets is carried out in two ways: wet granulation mixing or dry mixing.

Wet granulation mixing generally comprises the following steps:
(A) mixing the pharmaceutically active ingredients and the excipients to form a dry blend;
(B) moistening the dry blend with water, preferably purified water, to form a wet granulation blend;
(C) drying the wet granulation blend to form a dry granulation blend;
(D) milling the dried granulation blend to form a milled granulation blend;
(E) mixing a lubricant into the milled granulation blend to give a final blend;
(F) compress the final blend into tablets of a shape suitable for oral administration.

Descriptively in step (A), the pharmaceutical active ingredients are mixed with all excipients in the final formulation, except the lubricant, to form a uniform dry blend. Suitable mixers for large scale mixing include V-shaped, double cone, sigma Z, planetary and other mixers can also be used such as high speed, high shear mixers and offer the advantage of shorter mixing times. The dried blend may also be granulated, ground to a fine powder, passed through a mesh or micronized if necessary. Preferably, dry blending should be performed in high shear granulators.

The resulting dry blend is, then, moistened with a wetting agent to form a wet granulation blend in step (B). The wetting agent is added over time until it forms a homogeneous blend. Typically, the wetting agent is added to the mixer used in the dry blending step. Preferably the wet granulation is carried out in a large granulator. Preferably, the wetting agent is water without any additional solvents and, in particular, without organic solvents. More preferably, the water is purified water.

The type and the amount of wetting agent, the addition rate and mixing time influence the structure of the granules. The different types of granules, such as pendular, funicular, capillary, etc., can be manipulated to obtain the desired density, porosity, texture and dissolution pattern of the granules, which in turn determines the compressibility, hardness, disintegration and consolidation of the final dry blend.

The wet granulation blend is, then, dried in step (C) to form a dry granulation blend with an appropriate moisture content. In certain embodiments, the drying means includes a tray or fluid bed dryer. Fluid bed drying produces shorter drying times, while tray drying is longer. Preferably, the wet granulation blend is dried in a fluidized bed.

Fluid bed drying has the added advantages of better temperature control and reduced costs. Drying method, drying time and moisture content are critical to avoid decomposition, chemical migration and other adverse physical characteristics of the final dry mix that may affect tablet quality.

The dried granulation blend is subsequently milled in step (D) to form a milled granulation blend. The particle size of the dry granulation blend is reduced to obtain an appropriate particle size distribution for subsequent processes. In certain embodiments, milling is accomplished using a high impact shear mill (for example, Fitzpatrick) or a low shear screening mill (for example, Comil). The dried granulation blend can also be sieved to select the desired granule size.

In step (E), the lubricant is mixed with the dry granulation blend to give a final blend. In certain processes, V or bin mixer mixers are used. A preferred mixer is a PK V-shell mixer. A gentle blend is preferred so that each granule is covered with lubricant while minimizing granule breakage. Increased breakage of the granules results in fine powder. High dust content results in weight and density variations during tablet compression, as does the need for cleaning of the compression machine.

The final blend is then transferred to a compression machine typically containing two oblong steel punches within a steel die cavity. The tablet is formed when pressure is exerted on the dried granulation blend by the punches in the cavity or cell. Tablet making machines include single punch machines, rotary tablet machines, gravity feed and powder assisted machines. High-speed rotary machines suitable for large-scale production include double rotary machines and single rotary machines.

The tablets resulting from the process disclosed must present the characterized specifications of the official compendia including: shape, thickness, weight, weight variation, hardness, friability, disintegration time, dissolution time, content uniformity, quantification of active pharmaceutical ingredients, microbiological control and stability.

Depending on the use of different types of equipment employed in the described process, the expert in the art of pharmaceutical technology needs to perform small process variations in order to obtain tablets that meet the standards described in the official compendia following the good manufacturing practices defined for this process.

The direct compression dry blend comprises, in general, the following steps:
(A) mixing the pharmaceutically active ingredients with the excipients to form a dry blend;
(B) mixing a lubricant into the dry blend to give a final blend;
(C) compress the final blend into oblong shaped tablets suitable for oral administration.

Descriptively in step (A), the pharmaceutically active ingredients are mixed with all excipients in the final formulation except the lubricant. Suitable mixers for dry large-scale mixing include double shell mixers, double cone mixers, V-blenders or container mixers. A preferred mixer is a PK V-shell mixer. High speed and high shear mixers may also be used. The dried blend may also be ground to a fine powder, passed through a specific mesh or micronized if necessary.

In step (B), the lubricant is mixed with the dry blend to give a final blend. In certain embodiments, V or bin mixer mixers are used. A preferred mixer is a PK V-shell mixer.

The final blend is, then, transferred to a compression machine containing, typically, two oblong steel punches within a steel die cavity. The tablet is formed when pressure is exerted on the dry granulation blend by the punches in the cavity or cell. Tablet making machines include single punch machines, rotary tablet machines, gravity feed and powder assisted machines. High-speed rotary machines suitable for large-scale production include double rotary machines and single rotary machines.

The tablets resulting from the process disclosed must present the characterized specifications of the official compendia including: shape, thickness, weight, weight variation, hardness, friability, disintegration time, dissolution time, humidity, content uniformity, quantification of active pharmaceutical ingredients, microbiological control and stability.

Depending on the use of different types of equipment employed in the described process, the expert in the art of pharmaceutical technology needs to perform small process variations in order to obtain tablets that meet the standards described in the official compendia following the good manufacturing practices defined for this process.

Other alternative pharmaceutical form used is the product in the form of coated tablets. The tablets are coated with a film of several polymeric substances which perform several functions, for example: they allow to resist gastric juice, but disintegrate in the intestine releasing the active pharmaceutical ingredients; mask the possible odor and taste; promote greater tablet protection by increasing the stability of active inputs; facilitate administration; make tablets tougher.

There are several compositions of the coating solution available, being the most common enteric coatings, that is, they do not dissolve in the stomach acid medium, but dissolve when at a pH higher than 5.

Polymers used in the coating include: cellulose acetate phthalate, polyvinyl acetate phthalate, acrylic copolymer, methacrylate, polyvinyl alcohol polyethylene glycol copolymer; using the plasticizers: glycerin, propylene glycol, glycols; added colorant and flavoring substances in volatile organic solvents.

Coating film can be applied in a variety of ways, from manual application to sophisticated high production equipment available on the market (Nicomed, Pelegrini, IMA). In general, the tablets move constantly, and the coating solution is atomized on its surface, depositing the polymer in thin layers. The solvent is evaporated and exhausted from the system. In the manual process a circular pan coating is used containing the tablets where solution is atomized with a tablet air gun. In the present composition, if preferably the polymethacrylic acid / methyl methacrylate solution 1/1 91.

The coated tablets were analyzed in the whole productive process, mainly as regards its aspect and weight, according to the official compendia.

For significant results to be reported, the tablets should be taken three times a day, together with the meals, for adult individuals, three times a day for children between 7 and 10 years old and three times a day for children between 4 and 6 years old.

In another alternative pharmaceutical form, the compound is presented in the form of hard gelatin capsules, which is a usual method of presentation preferred by many patients who find it easier to swallow as well as tasteless. It is a process of easy extemporaneous production or large scale.

The capsules consist of two sections, in which one slides over the other, totally encompassing the formulation within. The capsule is filled with the composition at its longest bottom or the body of the capsule and then closed with the top or lid. There are various capsule filling machines, which are available in the market in various sizes ranging from capsule capacity or volume from 000 to 5, these can be manual or high production vacuum or compression filling machines.

In this alternative, the capsules were produced by the process of filling with compacting feeder, using the same direct compression dry blend previously disclosed, with a weight per capsule of 1100,0 mg using capsules no. 00. The production was performed in humidity-controlled environment and was controlled throughout the process for weight uniformity. The capsules produced are clean from dust generated in the filling.

The capsules must present the characterized specifications of the official compendia including: shape, weight, weight variation, disintegration time, dissolution time, humidity, content uniformity, quantification of active pharmaceutical ingredients, microbiological control and stability.

For significant results to be reported, the capsules should be taken three times a day and together with the meals, for adult individuals, three times a day for children between 7 and 10 years old and three times a day for children between 4 and 6 years old.

Another alternative pharmaceutical form is presented in soft gelatin capsule. In this process, the capsules are manufactured and filled with active pharmaceutical ingredients at the same time.

The capsule shell is manufactured in an area with special temperature conditions and relative humidity of around 20 %, using reactors which dissolve under heating the film components which, in this presentation, consisted of gelatin, sorbitol, polyethylene glycol, preservatives such as methylparaben and propylparaben, yellow colorant FD & C no. 6 and titanium dioxide. The resulting blend was milled and deaerated to form a homogeneous solution. This blend was transferred to the machine forming a film then set to the appropriate thickness.

The film went through oil bath rolls, falling between the pan and the die rolls. The solution of the formulated composition was then accurately dosed through the metering piston in the matrix and the capsules were then mechanically closed on the film. The capsules were then washed with naphtha to remove the lubricating oil and finally dried.

The soft gelatinous capsules (globules) composition was formulated with the following amounts: Thiamine hydrochloride 200 mg, Magnesium Glycinate 50 mg, L-Theanine 100 mg, methyl cobalamin 0,5 mg, Lysine Hydrochloride 75 mg, ascorbic acid 150 mg, dissolved in sufficient amounts of excipients solution to 1 mL containing a blend of alcohol / water (85 / 15), castor oil 35, oleic acid, gelatin, glycerin and propyl paraben. Similar to the manufacture of the film, the composition was performed in a suitable reactor and in environment with controlled temperature and relative humidity.

The whole process was controlled according to current good manufacturing practice standards and, at last, the tablets were analyzed according to specifications established by official compendia.

Other high production processes must be used with the same composition through automatic equipment available in the market.

For significant results to be reported, the capsules should be taken three times a day and together with the meals, for adult individuals, three times a day for children between 7 and 10 years old and three times a day for children between 4 and 6 years old.

Another alternative pharmaceutical form was performed through effervescent tablets. The raw materials present in effervescent formulations have the ability to buffer the solution where they are and, consequently, raise the gastric pH upon intake of the effervescent solution. On the other hand, this buffering effect induces faster stomach emptying, which will maximize absorption of the active pharmaceutical ingredient at the intestine level.

An example of effervescent tablets formulation for the composition of the present invention is the following:

| | |
|---|---|
| Thiamine hydrochloride | 400,0 mg |
| Magnesium Glycinate | 100,0 mg |
| L-Theanine | 200,0 mg |
| Methyl cobalamin | 1,0 mg |
| Lysine Hydrochloride | 150,0 mg |
| Ascorbic acid | 300,0 mg |
| Tartaric acid | 350,0 mg |
| Sodium bicarbonate | 450,0 mg |
| Orange flavor | 19,0 mg |
| Polyvinylpyrrolidone | 30,0 mg |
| Saccharin | 3,0 mg |
| Sodium cyclamate | 7,0 mg |
| Polyethylene glycol 6000 | 50,0 mg |
| Yellow colorant FD & C no. 6 | 20,0 mg |
| Sugar | 420,0 mg |
| Total | 2500 mg |

### Production process

In a double-bladed mixer, Thiamine hydrochloride, Magnesium Glycinate, L-leucine, Lysine Hydrochloride, Tartaric acid, Sodium bicarbonate, sugar and the polyvinylpyrrolidone were mixed. The blend was granulated with the help of isopropylic alcohol and dried. The dry blend was homogenized through a 0.8 mm mesh sieve.

To the homogeneous granulate obtained, it was added methyl cobalamin, ascorbic acid, orange flavor, saccharin, cyclamate, colorant and polyethylene glycol. The final blend was, then, compressed on rotary machines with 20 mm diameter punches and weighing 2,5 g.

The whole process was performed in controlled atmosphere with maximum relative humidity of 30 %, since the effervescent tablets must have at most 0.5 % residual humidity to maintain stability. Thus, the tablets produced were packaged in the same environmental conditions immediately after production in packaging, preferably in aluminum or plastic, maintaining the tablets dry and protecting them from light and oxygen.

The whole process was controlled according to current good manufacturing practice standards and, at last, the tablets were analyzed according to specifications established by official compendia.

For significant results to be reported, the effervescent tablets should be taken three times a day and together with the meals, in adult individuals, three times a day for children from 7 to 10 years old and three times a day for children from 4 to 6 years old.

Another example of pharmaceutical form for the present composition is performed through chewing gum, presenting the following composition:

| | |
|---|---|
| Thiamine hydrochloride | 400,0 mg |
| Magnesium Glycinate | 100,0 mg |
| L-Theanine | 200,0 mg |
| Methyl cobalamin | 1,0 mg |
| Lysine Hydrochloride | 150,0 mg |
| Ascorbic acid | 300,0 mg |
| Yellow colorant FD & C no. 6 | 20,0 mg |
| Glucose syrup | 337,0 mg |
| Orange flavor | 29,0 mg |
| Methyl paraben | 3,0 mg |
| Propylparaben | 10,0 mg |
| Gum base | 950,0 mg |

### Production process:

The base gum is composed of the following components: elastomers (butadiene styrene, polyvinyl acetate, polyisobutylene, polyisoprene and natural latex); resin; elastomers solvent (ester gum - Breu and terpenic resins); non sticking agents (paraffins, microcrystalline waxes, beeswax, carnauba wax, polyvinyl acetate and others); emulsifiers and / or plasticizers (glycerin monostearate, soy lecithin, triacetin, hydrogenated vegetable fats and others); texturizers (calcium carbonate and talc); antioxidants and conservatives (butylated hydroxy anisole (BHA), butylated hydroxy toluene (BHT), potassium sorbate), aromas.

Traditional chewing gum manufacture involves the following steps: mixing, extruding, cooling, molding and packaging.

The equipment used to manufacture the chewing gum was a Double Sigma or Double "Z" type, which is suitable for high viscosity material blends: two blades inserted in a pan, each in letter "Z" format and which may have tangential or overlapping action. The arm profile ensures perfect product dispersion in a shorter period of time. The two blades are in opposite directions for each layer during phase mixing. The mixer is characterized by its very robust construction and has a heating jacket that can act with oil, water or steam. Base gum components were added at a temperature between 45 and 55 °C and mixed with the active ingredients in the amounts described in the formula, added with the colorant, aroma and glucose syrup, until a homogeneous mass is formed.

The mass was, then, discharged from the mixer and placed in an extruder, forming the strands that were cooled and are going to be molded. The extruder temperature was controlled and maintained at approximately 45 °C to ensure that the mass did not cool and that the strands were well formed.

The strands formed at the extruder outlet were continuously transferred to a cooling tunnel. The internal temperature of the tunnel was also controlled to 20 °C. The dwell time in the tunnel was 20 minutes. Relative humidity was also controlled not exceeding the 65 %.

The cooled strands were, then, molded in unities weighting 2,5 g and packed in aluminized paper that will protect them from moisture and provide greater stability.

The whole process was controlled according to the current good manufacturing practice standards and, at last, the tablets were analyzed according to specifications established by official compendia.

For significant results to be reported, chewing a gum three times a day, to adult individuals, three times a day to children between 7 and 10 years old.

Another example of pharmaceutical form used for the present composition is a transdermal patch, presenting the composition, which presents single dose:

| | |
|---|---|
| Thiamine hydrochloride | 20,0 mg |
| Magnesium Glycinate | 5,0 mg |
| L-Theanine | 10,0 mg |
| Methyl cobalamin | 0,1 mg |
| L-Lysine Hydrochloride | 7,5 mg |
| Ascorbic acid | 14,9 mg |
| Sufficient base for 2 cm² patches | 52,5 mg |
| TOTAL | 110 mg |

The transdermal patches, when applied to the skin, release the active pharmaceutical ingredients that, after crossing the various layers of the skin, reach the bloodstream at a constant rate during the administration period. Transdermal patches generally have an outer membrane, a reservoir with active ingredients, a porous membrane, an adhesive layer and a disposable film. Such patches are generally applied to areas of increased blood flow and constant thickness of the skin, such as the upper arm or chest.

The transdermal patches have, as main advantages, the direct reach in the bloodstream, the application in different places of the body, besides increasing the patient's adherence to the treatment due to the ease of administration and decreased systemic toxicity, providing adequate absorption and controlled active plasma levels, besides have a lower frequency of administration and yet avoid risks from other administrations by removing them.

The composition herein claimed was constituted by a "patch coated layer" and refers to the surface wherein the composition was applied, and which will be in contact with the skin. The compounds of the patch coated layer disclosed in this specification with reference to the surface of the skin in which the composition should be applied (1 cm² and 2 cm²).

The most distal layer of the composition is a support layer which is flexibly fit to the surface of the skin using, as material, an ethyl vinyl acetate polymeric film. The support layer is waterproof and contain pores in the area, which is in contact with the skin, using patch like administration in which the active pharmaceutical ingredients can be found on the coated layer.

The film coating is a removable and disposable element serving only to protect the transdermal device prior to application to the skin. Typically, the removable coating is formed of a material impermeable to the active ingredient.

The base was formulated in specific equipment containing 20 % of tween 80, 5 % of isobutanol, 5 % of pyrrolidone, 30 % of isopropyl myristate, 20 % of span 20 and 20 % of water.

### Production process

Firstly, the ingredients were prepared and intensively mixed to obtain a uniform and homogeneous distribution of the active pharmaceutical ingredients at all points of the patch. The blend was secured to the patch backing layer in predetermined sizes and dried. After this, the outer coating layer was applied and finally cut and packed.

The whole process was controlled according to the norms of good manufacturing practices and, at last, the patches were analyzed according to the specifications established by the official compendia.

There are several types of transdermal patches preparations possible of being used as devices using the reservoir system, matrix systems and micro and multi-layer reservoirs.

To present significant results, a 2 cm² patch should be applied every 24 hours to adult individuals and a 1 cm² patch should be applied every 24 hours to children from 7 to 10 years.

The present invention has been disclosed in this description in terms of its preferred embodiment. However, other modifications and variations are possible from the present disclosure and are still within the scope of the invention herein disclosed.

## Claims

1. Multivitamin composition **characterized by** the fact that it comprises the following formulation: from 17 % to 35 % of Vitamin B1 or Thiamine Hydrochloride, from 5 % to 9 % of Magnesium or Magnesium Glycinate, from 8 % to 17 % of Lysine or Lysine Hydrochloride, from 4 % to 13 % of Theanine or L-Theanine, from 8 % to 26 % of Vitamin C or Ascorbic Acid, from 0,05 % to 0,9 % of Vitamin B12 or Methyl cobalamin and excipients.

2. Multivitamin composition, according to claim 1, **characterized by** the fact that it comprises active ingredients from 40 % to 70 % of the total of the composition and excipients from 30 % to 60 % of the total of the composition.

3. Multivitamin composition, according to any of claims 1 or 2, **characterized by** the fact that the excipients used comprise diluting agents, binding agents, disintegrating agents, lubricants, binders, non-sticking agents, gliding agents, wetting agents and surfactants, colorant and pigments, flavoring agents, sweeteners and flavor masks, but not limited to the same.

4. Multivitamin composition, according to any of claims 1 to 3, **characterized by** the fact that it comprises one or more of diluting agents, selected from starch and its derivatives, lactose monohydrate, microcrystalline cellulose, lactose and its derivatives, such as spray dried monohydrated lactose, alpha-lactose monohydrate, anhydrous alpha-lactose, anhydrous beta-lactose and agglomerated lactose, sugars, dextrose, inorganic salts, such as dibasic calcium phosphate, tribasic calcium phosphate, calcium sulfate, polyols such as mannitol, sorbitol and xylitol, but not limited to the same.

5. Multivitamin composition, according to any of claims 1 to 4, **characterized by** the fact that it comprises one or more of binding agents, selected from microcrystalline cellulose, methylcellulose, sodium carboxy methylcellulose, hydroxypropyl methylcellulose, hydroxyethyl cellulose and hydroxypropyl cellulose, Povidone K-29/32 and polyvinylpyrrolidone, but not limited to the same.

6. Multivitamin composition, according to any of claims 1 to 5, **characterized by** the fact that it comprises one or more of binding agents, selected from polyvidone, polyvinylpyrrolidone, gelatin, natural gums, such as acacia, tragacanthin, guar and pectin, starch pulp, pregelatinized starch, sucrose, corn syrup, polyethylene glycols and sodium alginate, calcium and ammonium alginate, magnesium and aluminum silicate, polyethylene glycols, Povidone and Povidone K-29/32, but not limited to the same.

7. Multivitamin composition, according to any of claims 1 to 6, **characterized by** the fact that it comprises one or more of lubricants agents, selected from plant oils, such as corn oil, mineral oils, polyethylene glycols, such as PEG-4000 and PEG-6000, salts of stearic acid, such as calcium stearate and sodium stearyl fumarate, mineral salts, inorganic salts, such as sodium chloride, organic salts, such as sodium benzoate, sodium acetate and sodium oleate and polyvinyl alcohols, calcium stearate and magnesium stearate, but not limited to the same.

8. Multivitamin composition, according to any of claims 1 to 7, **characterized by** the fact that it comprises one or more of disintegrating agents, selected from starch derivatives, such as cross-linked sodium salt of a starch carboxymethyl ether, such as sodium starch glycolate, pregelatinized starch, cross-linked sodium carboxymethyl cellulose, such as sodium croscarmellose, cross-linked polyvinylpyrrolidone, such as Crospovidone and the microcrystalline cellulose, but not limited to the same.

9. Multivitamin composition, according to any of claims 1 to 8, **characterized by** the fact that it comprises one or more of non-stick agents, selected from paraffins, microcrystalline waxes, beeswax, carnauba wax, polyvinyl acetate, but not limited to the same.

10. Multivitamin composition, according to any of claims 1 to 9, **characterized by** the fact that it comprises one or more of gliding agents, selected from talc and silicon dioxide, but not limited to the same.

11. Multivitamin composition, according to any of claims 1 to 10, **characterized by** the fact that it comprises one or more of wetting agents, selected from alcohol, water, propylene glycol and glycerin, but not limited to the same.

12. Multivitamin composition, according to any of claims 1 to 11, **characterized by** the fact that it comprises one or more of surfactants, selected from sodium lauryl sulfate and ammonium quaternary, but not limited to the same.

13. Multivitamin composition, according to any of claims 1 to 12, **characterized by** the fact that it comprises one or more of colorant and pigments, selected from the ones included in the list (FD & C) approved by the competent bodies, but not limited to the same.

14. Multivitamin composition, according to any of claims 1 to 13, **characterized by** the fact that it comprises one or more of flavoring, sweeteners and flavor masks, selected from orange flavor, glucose syrup, mannitol, lactose, sugar, but not limited to the same.

15. Multivitamin composition, according to any of claims 1 to 14, **characterized by** the fact that it is used in the improvement in verbal fluency and performance anxiety in adults and children and in the amelioration and relief of the physical, psychological and behavioral symptoms caused by them.

16. Multivitamin composition, according to any of claims 1 to 15, **characterized by** the fact that it is administered in single dose.

17. Multivitamin composition, according to claim 16, **characterized by** the fact that the transdermal patch pharmaceutical form is administered in single dose.

18. Multivitamin composition, according to any of claims 1 to 15, **characterized by** the fact that it is, preferably, administered in three daily doses.

19. Multivitamin composition, according to any of claims 1 to 18, **characterized by** the fact that it can be administered in the following pharmaceutical forms: tablet, chewing gum, transdermal patches, effervescent tablets, capsules, lozenges, solutions, suspensions, but not limited to the same.
